# EUROPEAN PATENT APPLICATION

(11) **EP 4 219 533 A1**
(43) Date of publication of application: **02.08.2023**
(21) Application number: 21872679.2
(22) Date of filing: 08.07.2021
(51) Int. Cl.: C07K 14/47, C07K 14/39, C12N 15/81, A61K 47/64, A61K 31/015, C12P 5/00

(54) **RECOMBINANT MICROORGANISM COMPRISING POLYNUCLEOTIDE ENCODING TARGET PRODUCT BINDING PROTEIN FUSED TO SECRETION SIGNAL SEQUENCE, COMPOSITION COMPRISING SAME, AND METHOD FOR PRODUCING TARGET PRODUCT BY USING SAME**

(30) Priority: 28.09.2020 KR 20200125985; 23.03.2021 KR 20210037445
(71) Applicant: Korea Research Institute of Chemical Technology, Daejeon 34114 (KR)
(72) Inventor: LEE, Ju Young, Daejeon 34114 (KR); SON, So Hee, Daejeon 34114 (KR); KIM, Jae Eung, Daejeon 34114 (KR)
(74) Representative: Mewburn Ellis LLP
(86) International application number: PCT/KR2021/008714
(87) International publication number: WO 2022/065643

(57) **Abstract**

Provided are a recombinant microorganism, a composition comprising the same, and a method of producing a target product by using the same, the recombinant microorganism comprising a polynucleotide encoding a target product-binding protein fused to a secretion signal sequence.

## Description

### Technical Field

The present disclosure relates to a recombinant microorganism including a polynucleotide encoding a target product-binding protein fused to a secretion signal sequence, a composition including the recombinant microorganism, and a method of producing a target product by using the recombinant microorganism.

### Background Art

Many products are produced by using recombinant microorganisms. For example, by using recombinant *E*. *coli* or yeast cells including *S*. *cerevisiae,* several products are produced.

However, some products are present in cells after being biosynthesized in recombinant microorganisms. Such products present in cells require an additional step of disrupting the cells to be isolated therefrom.

Therefore, there is a need for a recombinant microorganism capable of secreting a product present in a cell after being biosynthesized to the outside of the cell and for a method of producing a product by using the recombinant microorganism.

### Disclosure

### Technical Problem

One aspect provides a recombinant microorganism comprising a polynucleotide encoding a target product-binding protein fused to a secretion signal sequence.

Another aspect provides a composition for use in producing a target product, comprising a carrier and the recombinant microorganism comprising a polynucleotide encoding a target product-binding protein fused to a secretion signal sequence.

Another aspect provides a method of producing a target product, the method comprising culturing the recombinant microorganism in a medium, wherein the recombinant microorganism comprises a polynucleotide encoding a target product-binding protein fused to a secretion signal sequence.

### Technical Solution

One aspect provides a recombinant microorganism comprising a polynucleotide encoding a target product-binding protein fused to a secretion signal sequence.

The polynucleotide may be present in the genome or outside of the genome of the recombinant microorganism. The polynucleotide may be operably linked to a regulatory sequence. The regulatory sequence may be a sequence necessary for biosynthesis or production of the protein from the polynucleotide. The regulatory sequence may be a transcriptional regulatory sequence, a translational regulatory sequence, and a post-translational modification regulatory sequence. The regulatory sequence may be a promoter, an operator, a terminator, an enhancer, a ribosomebinding site, or a combination thereof. The regulatory sequence may be endogenously present before the recombinant microorganism is recombined, or may be an exogenous regulatory sequence. The regulatory sequence may be a regulatory sequence of a gene encoding a naked target product-binding protein to which the secretion signal sequence is not fused, or may be a regulatory sequence of a gene encoding other proteins. The polynucleotide may be included in multiple copies in the cell. The polynucleotide may be, for example, included in copies in a range of 2 or more, 5 or more, 10 or more, 50 or more, or 100 or more copies or in a range of 2 to 1,000, 2 to 500, 2 to 100, 2 to 50, 2 to 10, 2 to 5, 5 to 100 , 5 to 50, 5 to 10, 5 to 100, 5 to 10, 10 to 100, or 10 to 50 in the cell. The polynucleotide may be linked to the regulatory sequence to enable expression. The polynucleotide may be linked to the regulatory sequence that allows expression at an increased level compared to an expression level of the endogenous polynucleotide. For example, the polynucleotide may be linked to a promoter having stronger ability for initiation of transcription than an endogenous promoter.

The polynucleotide may also include a nucleotide sequence encoding a tag sequence for use in isolating the complex or the dissociated target product-binding protein secreted extracellularly. The tag sequence may be an oligopeptide. The tag sequence may include a sequence of two or more amino acids, for example, 2 to 50, 2 to 30, 2 to 20, 2 to 15, or 2 to 10 amino acids. The tag sequence may be, for example, a His tag sequence. The His tag may be linked to the N-terminus or the C-terminus of the target product-binding protein. The tag may be 6xHis.

The polynucleotide may encode an amino acid sequence in which a secretion signal sequence is fused to the N-terminus of the target product-binding protein.

The polynucleotide may be introduced into a microbial cell before being recombined by a genetic modification method known in the art. The genetic modification may include transformation, transduction, transfection, or electroporation. In addition, the polynucleotide may be produced by gene editing of a gene in a microbial cell before being recombined. The gene editing may be performed by a known method in the art, such as clustered regularly interspaced short palindromic repeats (CRISPR) gene editing.

The recombinant microorganism may have an ability to secrete the target product-binding protein fused to the secretion signal sequence. The recombinant microorganism may have an ability to produce the target product. The recombinant microorganism may have an increased ability to produce the target product. The recombinant microorganism may be secreted extracellularly in the form of a complex in which the target product and the target product-binding protein are bound. Formation of the complex may be performed intracellularly. The formation of the complex may be achieved by cells in a cellular metabolic process without manipulation from the outside. In the complex, the bond between the target product and the target product-binding protein may be a non-covalent bond. The non-covalent bond may include a hydrophobic bond, a hydrogen bond, or an ionic bond. The complex may be dissociated in a liquid medium under conditions suitable for dissociating the bond to form a dissociated target product and the target product-binding protein, respectively. A target product-binding protein that is not fused to the secretion signal sequence may be present intracellularly, for example, in the cytoplasm of a natural host cell, and thus may not be secreted extracellularly. The target product-binding protein not fused to the secretion signal sequence may not be linked to the secretion signal sequence in the natural host cell.

The target product may be a metabolite. The metabolite may be an endogenous metabolite or a metabolite synthesized by a synthesis pathway thereof introduced by genetic modification. The metabolite may be a lipid, a polysaccharide, a protein, or a small molecule compound. The target product may be a lipid. The lipid may be a terpenoid. The term "terpenoid" as used herein refers to a compound synthesized by using isoprene as a unit. The number of carbon atoms in the terpenoid may be in a range of 5 to 100, 10 to 100, 15 to 100, 30 to 100, 5 to 60, 10 to 60, 15 to 60, 30 to 60, 5 to 45, 10 to 45, 15 to 45 carbon atoms , 30 to 60, 5 to 30, 10 to 30, 15 to 30, or 5 to 45. The terpenoid may include oxygen molecules. In addition, the terpenoid may be a hydrocarbon that does not contain oxygen molecules. The terpenoid may be monoterpene, diterpene, sesquiterpene, triterpene, or tetraterpene. The terpenoid may be a cyclic compound or a non-cyclic compound. The terpenoid may be selected from the following 43 compounds, i.e., 2,3-oxidosqualene, myrcene, (z)-ocimene, linalool, geraniol, nerol, limonene, menthol, α-pinene, camphor, carvacrol, carvone, β-farnesene, nerolidol, β-bisabolene, valencene, amorphadiene, capsidiol, α-cedrane, artemisinin, phytane, phytol, camphorene, retinol, labdane, clerodane, pimarane, abietane, tigliane, kaurane, taxane, squalene, lanosterol, α-amyrin, β-amyrin, hopane, oleanolic acid, lycopene, rubixanthin, β-carotene, lutein, zeaxanthin, and astaxanthin. The target product may be squalene or beta-carotene. The recombinant microorganism may have an enhanced biosynthetic pathway of terpenoid. The recombinant microorganism may have an enhanced biosynthetic pathway of one or more of the 43 compounds.

The target product-binding protein may bind to the metabolite. The target product-binding protein may specifically bind to the metabolite. The target product-binding protein may be a lipid-binding protein. The target product-binding protein may be a cytoplasmic lipid-binding protein. The lipid-binding protein may be selected from the group consisting of a supernatant protein factor (SPF), a phosphatidylinositol transfer protein (Sec14), an alpha-tocopherol transfer protein (TTP), a cellular retinal binding protein, a squid retinal binding protein, and fragments thereof having target product-binding ability. The lipid-binding protein may be derived from any cell. For example, the lipid-binding protein may be derived from a mammalian cell. The SPF and the TTP may be derived from a human or a mouse. The human-derived SPF has a sequence of 403 amino acids , and may have an N-terminal domain with lipid binding ability and a C-terminal domain with a jelly-roll motif, i.e., a golgi dynamics domain (GOLD). The SPF may be a truncated SPF (tSPF) having squalene binding ability and a C-terminal domain in which the amino acid sequences at positions 276 to 403 have been removed by cleavage. That is, the tSPF may consist of the sequence of amino acids 1 to 275 of the SPF. The SPF and the tSPF may have amino acid sequences of SEQ ID NOs: 1 and 2, respectively. Nucleotides encoding the SPF and the tSPF may have nucleotide sequences of SEQ ID NOs: 3 and 4, respectively.

The secretion signal sequence represents an amino acid sequence that secretes a protein expressed in a cell to the outside of the cell. The secretion signal sequence may be a secretion signal sequence derived from a eukaryotic cell or a prokaryotic cell. The secretion signal sequence may be any amino acid sequence that can secrete the target product-binding protein from the recombinant microorganism to the outside of the cell. The secretion signal sequence may be derived from the same or different cell from the recombinant microbial cell. The secretion signal sequence may be a secretion signal sequence derived from animals, plants, yeast, or bacteria. The secretion signal sequence may be one or more selected from the group consisting of Suc2, Pho5, MFα, Inu1, and Mel1 secretion signal sequences. The Suc2, Pho5, and MFα secretion signal sequences may have amino acid sequences of SEQ ID NOs: 5, 6, and 7, respectively. Nucleotides encoding the Suc2, Pho5, and MFα secretion signal sequences may have nucleotide sequences of SEQ ID NOs: 8, 9, and 10, respectively.

The recombinant microorganism may be a eukaryotic cell or a prokaryotic cell. The eukaryotic cell may be an animal cell, a plant cell, or a fungal cell. The fungus may be yeast. The yeast cell may belong to the genus *Saccharomyces.* A cell of the genus *Saccharomyces* may be *Saccharomyces cerevisiae.* The prokaryotic cell may be a bacterium. The bacteria may belong to the genus *Escherichia,* the genus *Corynebacterium,* or the genus *Bacillus.* In addition, the bacteria may be lactic acid bacteria belonging to, for example, the genus *Lactobacillus* or the genus *Lactococcus.* The bacteria may be *Escherichia coli, Corynebacterium glutamicum,* or *Bacillus subtilus.*

The recombinant microorganism may be genetically modified to increase the expression of the polynucleotide encoding the target product, or may have an enhanced metabolic pathway for producing the target product. The recombinant microorganism may have an increased ability to produce the target product compared to cells before transformation. The genetic modification may include an increase in the number of copies of the polynucleotide encoding the target product. The genetic modification may include modification of the regulatory sequence to increase the expression of the polynucleotide encoding the target product. In addition, the recombinant microorganism may have an enhanced metabolic pathway for producing the target product. The metabolic pathway for producing the target product may be enhanced by genetic modification that increases the expression of the polynucleotide encoding a factor, such as a protein or an enzyme, involved in the biosynthesis of the target product. The genetic modification may include an increase in the number of copies of the polynucleotide encoding a factor, such as a protein or an enzyme, involved in the biosynthesis of the target product, or modification of the regulatory sequence of the polynucleotide.

The recombinant microorganism may also have an attenuated metabolic pathway that inhibits the production of the target product. The metabolic pathway may be, for example, a pathway that degrades the target product or a feedback inhibition pathway.

In an embodiment, the target product-binding protein may be SPF or a ligand binding fragment thereof. The target product-binding protein may be a polypeptide including amino acids, such as L84, 1103, L106, A108, L111, L112, L120, L121, K124, 1151, Y153, C155, L158, H162, A167, V168, A170, Y171, F174, L175, L186, L189, F198, A201, Y202, I205, L209, T213, and I217 in the amino acid sequence of the SPF of SEQ ID NO: 1. The recombinant microorganism may belong to the genus *Saccharomyces,* and for example, may be *Saccharomyces cerevisiae.* The recombinant microorganism may have an enhanced biosynthesis pathway of mevalonic acid or beta-carotene. The recombinant microorganism may include a genetic modification that increases activity of one or more of HMG-CoA and ERG20. The recombinant microorganism may include a genetic modification that increases expression of a gene encoding HMG-CoA or ERG20. The genetic modification may include an increase in the number of copies of the gene. In the recombinant microorganism, one or more of trp1, leu2, his3, and yp1062w genes may be inactivated. The genetic modification may include not only completely eliminating but also reducing the expression of the gene to be inactivated. The inactivation may be deletion of one or more of the trp1, leu2, his3 and yp1062w genes. HMG-CoA, ERG20, trp1, leu2, his3, and yp1062w may have amino acid sequences of SEQ ID NOs: 11, 12, 13, 14, 15, and 16, respectively. HMG-CoA, ERG20, trp1 gene, leu2 gene, his3 gene, and yp1062w gene may have nucleotide sequences of SEQ ID NOs: 17, 18, 19, 20, 21, and 22, respectively.

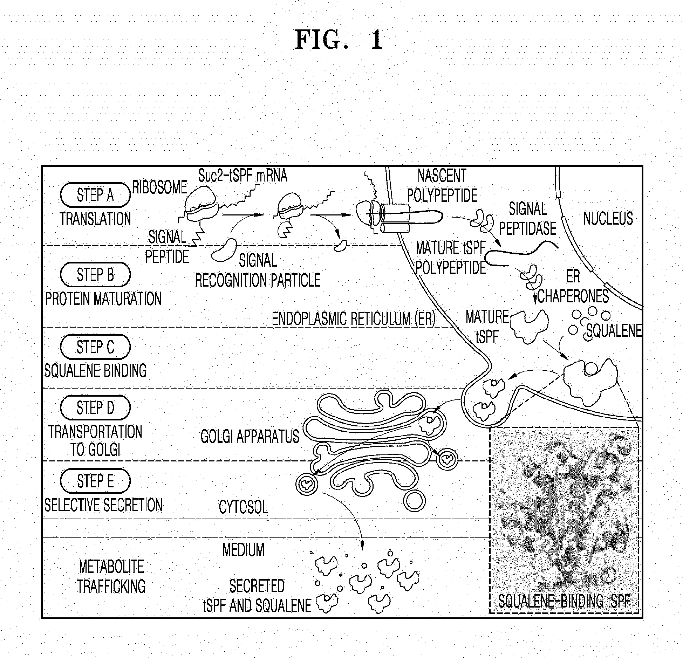

FIG. 1 is a diagram schematically showing a process of squalene secretion in yeast cells containing a polynucleotide encoding a Suc2-tSPF fusion protein. In this process, first, during translation of mRNA transcribed from the polynucleotide in the cell, a signal peptide located at the N-terminus is bound to a signal recognition particle. Then, a complex of the partially translated fusion protein and a ribosome is transported to the endoplasmic reticulum membrane. When a nascent polypeptide of the fusion protein is synthesized in the endoplasmic reticulum, and the signal peptide is cleaved by a signal peptidase in the lumen of the endoplasmic reticulum, only tSPF, that is, a polypeptide of a target product-binding protein remains (step A). The polypeptide of the binding protein may undergo further modification by a chaperone protein of the endoplasmic reticulum, and then may form a mature protein (step B). The mature squalene-specific binding protein may form a complex by specifically binding with squalene accumulated in the cell. This process is the process of capturing the target metabolite (step C). When squalene forms a complex with the binding protein, the complex encapsulated by a transport vesicle can be transported to the golgi apparatus (step D). The complex of squalene and the binding proteins can be released into the extracellular space by a secretory vesicle from the golgi apparatus (step E).

Another aspect provides a composition for use in producing a target product, the composition comprising a carrier and the recombinant microorganism comprising a polynucleotide encoding a target product-binding protein fused to a secretion signal sequence.

The recombinant microorganism comprising a polynucleotide encoding a target product-binding protein fused to a secretion signal sequence is defined the same as described above. The carrier may be a medium used for culturing the microorganism, a buffer, or a storage solution.

Another aspect provides a method of producing a target product, the method comprising culturing the recombinant microorganism in a medium, wherein the recombinant microorganism comprises a polynucleotide encoding a target product-binding protein fused to a secretion signal sequence.

The recombinant microorganism comprising a polynucleotide encoding a target product-binding protein fused to a secretion signal sequence is defined the same as described above.

The culturing may include incubating a mixture including the recombinant microorganism and a medium under conditions suitable for growing the recombinant microorganism and producing a target product-binding protein fused to the secretory signal sequence.

The culturing may be performed in a medium containing a carbon source, for example, glucose. The medium used for culturing the microorganisms may be any conventional medium suitable for the growth of the selected recombinant cells, such as a minimal or complex medium containing appropriate supplements. Such a suitable medium may be available from commercial sellers, or can be prepared according to known methods.

The medium may be a medium that can satisfy the requirements of specific microorganisms according to the selected target product. The medium may include a component selected from the group consisting of a carbon source, a nitrogen source, a salt, a trace element, and a combination thereof.

Conditions of the culture may be appropriately adjusted to suit the production of a selected target product, for example, terpenoid such as squalene. The culturing may be performed under aerobic conditions for cell proliferation. The culturing may be performed without stirring or with stirring. The culturing may be performed in a batch or a fed batch, or continuously. The continuous culturing may refer to continuous culturing while not only removing some of the microorganism or supernatant from the culture, but also supplementing a medium. The recombinant microorganism may secrete the target product extracellularly in the form of a complex with the target product-binding protein, and that is, may be separated from the supernatant from which the cells are removed from the culture.

The culturing may refer to culturing in a medium containing a compound having high solubility of a target product. The solubility may be higher than that for the medium or water. The solubility for target product may be greater than 1.2, 1.5, 2.0, 3.0, 5.0, 7.5, or 10 times higher than the solubility for the medium or water, or may be 1.2 to 10, 1.5 to 10, 2.0 to 10, 3.0 to 10, 5.0 to 10, or 7.5 to 10 times higher than the solubility for the medium or water. The target product may be a lipid, such as terpenoid, and the compound having high solubility of the target product may be an oily compound having high solubility of the lipid, such as terpenoid. The target product may be secreted extracellularly in the form of a target product-target product-binding protein complex during culturing, and in the extracellular medium, the complex may exist in a state in which the complex is dissociated into the target product and the target product-binding protein and in a dynamic equilibrium state. Accordingly, a part of the target product dissociated in the dynamic equilibrium state may be distributed to a compound layer having high solubility of the target product. For example, when the target product is a lipid, such as terpenoid, and the culturing is performed in a medium containing an oily compound, the target product may be distributed to the oily compound in the medium after being secreted extracellularly. The oily compound may be an alkane solvent, for example, a C1-C60 alkane, a C6-C60 alkane, or a C3-C30 alkane. The solvent may be hexane, heptane, decane, dodecane, or hexadecane. In addition, the oily compound may be vegetable oil. The vegetable oil may be olive oil, canola oil, or essential oil. The oily compound may be a compound that is easy to separate from the target product due to a large boiling point difference from the target product.

The term "conditions for culture" as used herein refers to conditions for culturing microorganisms. Such conditions for culture may include, for example, carbon source, nitrogen source, or oxygen conditions used by microorganisms. A carbon source available to yeast may include monosaccharides, disaccharides, or polysaccharides. The carbon source is an assimilatable sugar, and may include glucose, fructose, mannose, or galactose. The nitrogen source may include an organic nitrogen compound or an inorganic nitrogen compound. The nitrogen source may include an amino acid, an amide, an amine, a nitrate, or an ammonium salt. Oxygen conditions for culturing microorganisms may include aerobic conditions with normal oxygen partial pressure, hypoxic conditions containing 0.1 % to 10 % oxygen in the atmosphere, or anaerobic conditions without oxygen.

The method may include isolating the target product. The isolating of the target product may not include cell lysis. The isolating of the target product may include separating a supernatant from a culture. The separating may include separating the target product from the supernatant. The isolating of the target product may include separating the target product from the complex of the target product and the target product-binding protein. The separating of the target product from the complex of the target product and the target product-binding protein may include incubating the culture or supernatant containing the complex in a liquid medium under conditions suitable for dissociating the complex into the target product and the target product-binding protein. The conditions may be appropriately selected depending on the selected target product and target product-binding protein. For example, the target product may be a terpenoid compound, such as squalene or beta-carotene, and the isolating of the target product may include incubating the complex in a hydrophobic solvent, for example, with an oily compound. The solvent may be an alkane, such as a C1-C60 alkane, a C6-C60 alkane, or a C3-C30 alkane. The solvent may be hexane, heptane, decane, dodecane, or hexadecane. The isolating of the target product may be performed simultaneously with the culturing of the recombinant microorganism.

In an embodiment, the target product may be a compound selected from the group consisting of 2,3-oxidosqualene, myrcene, (z)-ocimene, linalool, geraniol, nerol, limonene, menthol, α-pinene, camphor, carvacrol, carvone, β-farnesene, nerolidol, β-bisabolene, valencene, amorphadiene, capsidiol, α-cedrane, artemisinin, phytane, phytol, camphorene, retinol, labdane, clerodane, pimarane, abietane, tigliane, kaurane, taxane, squalene, lanosterol, α-amyrin, β-amyrin, hopane, oleanolic acid, lycopene, rubixanthin, β-carotene, lutein, zeaxanthin, and astaxanthin. The target product-binding protein may be selected from the group consisting of SPF, Sec14, TTP, an alpha-tocopherol transfer protein, a cellular retinal binding protein, a squid retinal binding protein, and fragments thereof having target product-binding ability. The recombinant microorganism may be a yeast cell, for example, of the genus *Saccharomyces.* The microorganism belonging to the genus *Saccharomyces* may be S. *cerevisiae.*

### ADVANTAGEOUS EFFECTS OF DISCLOSURE

A recombinant microorganism according to one aspect can be used to efficiently produce a target product.

A composition for use in producing a target product according to one aspect can be used to efficiently produce a target product.

According to a method of producing a target product according to one aspect, a target product can be efficiently produced.

### BRIEF DESCRIPTION OF DRAWINGS

FIG. 1 is a diagram schematically showing a process of squalene secretion in yeast cells containing a polynucleotide encoding a Suc2-tSPF fusion protein.
FIG. 2A is a diagram showing the concentrations of extracellular squalene produced by yeast cells containing a polynucleotide encoding tSPF fused to a secretion signal sequence.
FIG. 2B is a diagram showing the concentrations of intracellular squalene produced by yeast cells containing a polynucleotide encoding tSPF fused to a secretion signal sequence.
FIG. 3 is a diagram showing the results of HPLC analysis of squalene extracellularly secreted by SQ strains each transformed with Suc2-tSPF, Pho5-tSPF and MFα-tSPF fusion protein genes.
FIG. 4 is a diagram showing the results of western blot analysis of cell lysates and a culture supernatant after culturing SQ strains each transformed with Suc2-tSPF, Pho5-tSPF and MFα-tSPF fusion protein genes.

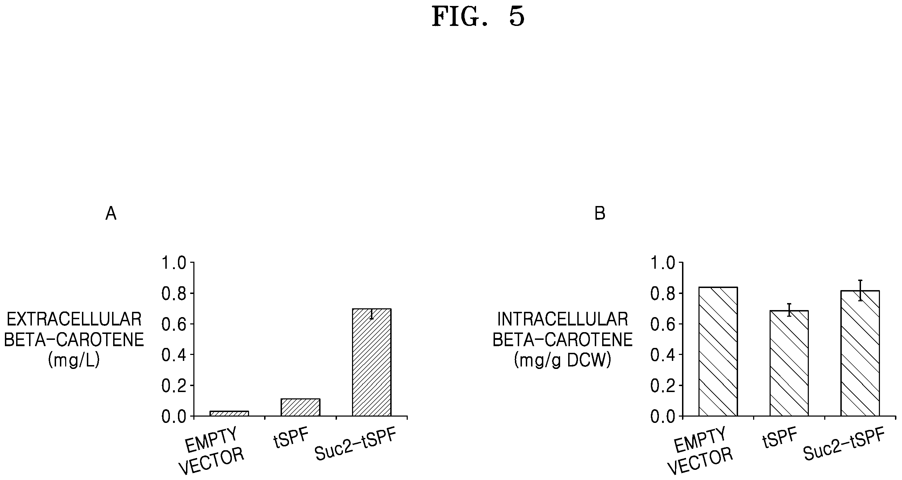

FIG. 5 shows the amount of beta-carotene produced by culturing CEN.PK2-1D yeast cells co-transformed with a p415-BC vector and either pSEC-tSPF or pSEC-Suc2-tSPF.
FIG. 6 is a diagram showing the results of HPLC analysis of beta-carotene produced by yeast cells transformed with a Suc2-tSPF fusion protein gene and then secreted extracellularly.

### BEST MODE

Hereinafter, the present disclosure will be described in detail with reference to Examples below. However, these Examples are for illustrative purposes only, and the scope of the present disclosure is not intended to be limited by these Examples.

### Example 1. Yeast cells with increased squalene secretion ability and squalene production by using the same

In this example, recombinant yeast cells were prepared by introducing a target product-binding protein fused to a secretion signal sequence into yeast cells, and a target product was produced by using the prepared recombinant yeast cells, and the secretion ability of the target product was confirmed. *Saccharomyces cerevisiae* was used as the yeast cell, and Suc2, Pho5, and MFα secretion signal sequences were used as secretion signal sequences, respectively. In addition, squalene was used as the target product, and tSPF, which is a truncated fragment of supernatant protein factor (SPF), was used as the target product-binding protein. tSPF is a squalene-binding protein corresponding to the N-terminal domain of SPF with lipid-binding ability. tSPF corresponds to amino acid sequences 1 to 275 in SPF having the amino acid sequence of SEQ ID NO: 1.

### (1.1) Preparation of yeast cells with enhanced biosynthetic pathway of squalene

Yeast cells were S. *cerevisiae,* and the wild-type strain was CEN.PK2-1D [(*MATα ura3-52; trp1-289; leu2-3, 112; his3△ 1; MAL2-8; SUC2)* (EUROSCARF Accession Number: 30000B). In the strain, genes encoding phosphoribosylanthranilate isomerase (trp1), 3-isopropylmalate dehydrogenase (leu2), and imidazoleglycerolphosphate dehydratase (his3) were inactivated. trp1, leu2, and his3 may have amino acid sequences of SEQ ID NOs: 13, 14, and 15, respectively. The genes encoding trp1, leu2, and his3 may have nucleotide sequences of SEQ ID NOs: 19, 20, and 21, respectively. Squalene is a terpenoid that is synthesized in yeast cells through a mevalonic acid biosynthetic pathway. To increase the squalene production ability, the following genetic modifications were introduced.

First, to the sites where the genes, i.e., *trp1, leu2,* and *his3,* were deleted from the strain, the tHMG1 gene encoding S. cerevisiae-derived HMG-CoA reductase (β-hydroxy β-methytgtutaryt-CoA reductase) and a gene encoding S. cerevisiae-derived farnesyl pyrophosphate synthetase (ERG20) were introduced. The tHMG1 and ERG20 may have amino acid sequences of SEQ ID NOs: 11 and 12, respectively. The genes encoding tHMG1 and ERG20 may have nucleotide sequences of SEQ ID NOs: 17 and 18, respectively. The HMG-CoA reductase is an enzyme that catalyzes the reaction of converting HMG-CoA to mevalonate. The ERG20 is an enzyme that catalyzes the conversion of GPP to farnesyl pyrophosphate (FPP). Both HMG-CoA and FPP may be precursors for the synthesis of squalene.

In addition, in the genome of the strain, the tHMG1 gene was introduced to the site of ypl062w while simultaneously deleting a gene encoding ypl062w. Due to the deletion of the ypl062w gene, the level of acetyl-CoA, which is an early substrate in the biosynthetic pathway of mevaloate, may increase. The ypl062w may have the amino acid sequence of SEQ ID NO: 16. The gene encoding ypl062w may have the nucleotide sequence of SEQ ID NO: 22.

As a result, a recombinant *S*. *cerevisiae* strain, named SQ, was obtained. A more detailed process for producing the SQ strain is disclosed in Korean patent KR 10-2176555, and the contents thereof are incorporated herein by reference.

### (1.2) Preparation of yeast cells to which gene encoding squalene-binding protein fused to secretion signal sequence has been introduced

Three different signal peptides were used for the secretion signal sequence, and tSPF was used for the squalene-binding protein. Specifically, a polynucleotide encoding a fusion protein was obtained, in which three different signal peptides, i.e., Suc2, Pho5, and MFα signal peptides, were each fused to the N-terminus of tSPF. The fusion protein may include a 6xHis tag sequence at the C-terminus.

The Suc2, Pho5, and MFα signal peptides may have the amino acid sequences of SEQ ID NOs: 5, 6, and 7, respectively. Nucleotides encoding the Suc2, Pho5, and MFα signal peptides may have nucleotide sequences of SEQ ID NOs: 8, 9, and 10, respectively. The Suc2, Pho5, and MFα signal peptides may be amino acid sequences with the function of N-terminal signal peptides, and may secrete sucrose transporter protein (Suc2), an acid phosphatase (Pho5), and a yeast α-mating factor (MFα), respectively, into the outside the cell. The tSPF may have the amino acid sequence of SEQ ID NO: 2. The tSPF-encoding gene may have the nucleotide sequence of SEQ ID NO: 4. As a result, the obtained fusion proteins, i.e., Suc2-tSPF, Pho5-tSPF, and MFα-tSPF fusion proteins, may have amino acid sequences of SEQ ID NOs: 23, 24, and 25, respectively. Nucleotides encoding the Suc2-tSPF, Pho5-tSPF, and MFα-tSPF fusion proteins may have nucleotide sequences of SEQ ID NOs: 26, 27, and 28, respectively.

Next, the polynucleotide was introduced into the Spel/Xhol restriction enzyme site of the p426-TEF1 (SEQ ID NO: 29) vector to obtain expression vectors pSEC-Suc2-tSPF, pSEC-Pho5-tSPF and pSEC-MFα-tSPF. As controls, the pSEC-tSPF vector to which the tSPF gene without the secretory signal sequence was introduced, and the empty p426-TEF1 vector were used. p426-TEF1 is a high copy number plasmid including a TEF1 promoter. The fusion protein is operably linked by the TEF1 promoter, and can be transcribed thereby.

The expression vectors were each introduced into the SQ strain by a heat shock transformation method to obtain SQ strains each transformed with three fusion protein genes. The expression vectors were present outside, independently from the genome of the SQ strain.

### (1.3) Confirmation of squalene production ability and squalene secretion ability by recombinant yeast cells

The SQ strains transformed with the pSEC-Suc2-tSPF, pSEC-Pho5-tSPF, and pSEC-MFα-tSPF expression vectors were cultured in flask (250 L) containing 50 mL of minimal medium (e.g., YSC medium; 0.67 % (w/v) yeast nitrogen base without amino acids (BD Difco, USA), 0.19 % (w/v) yeast synthetic drop-out medium supplements without uracil (Sigma-Aldrich, USA)) while stirring at 250 rpm at 30 °C.

After culturing for 72 hours and 144 hours, the culture was centrifuged at 4,000 rpm to be separated into cells, a culture supernatant, and a dodecane layer. Then, the concentration of squalene in the separated cells and dodecane layer was measured. To measure the squalene concentration in the cells, the cells were resuspended in 600 µL of a mixed solution containing methanol and acetone at 1:1, transferred to a tube containing lysing matrix C (glass bead, MP Biomedicals, USA), and then underwent cell lysis by using a FastPrep-24 5G homogenizer (MP Biomedicals, USA). By centrifugation at 13,000 rpm, the lysed cells and intracellular metabolites including squalene dissolved from the lysed cells were separated.

Next, the squalene concentration in the cell lysate was measured. To measure the squalene concentration in the cell lysate, the concentration was measured by using an Agilent HPLC system after filtering the cell lysate through a 0.2 µm syringe filter.

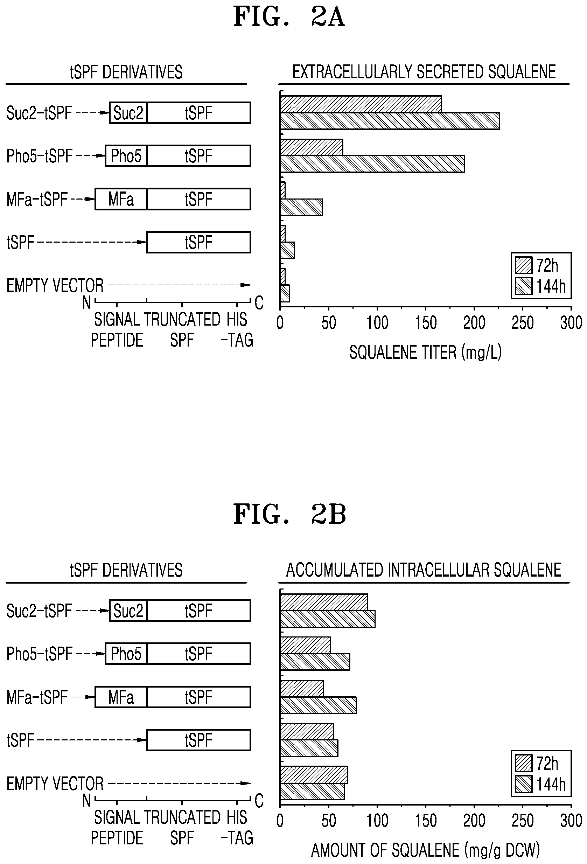

In addition, the dodecane layer separated by centrifugation was also filtered in the same way, and then the squalene concentration was measured by using an HPLC system. Table 1 shows the squalene concentration in the cells or outside the cells, wherein the cells were produced by yeast cells including the polynucleotide encoding tSPF fused to the secretion signal sequence. FIGS. 2A and 2B show the squalene concentrations outside the cells or in the cells, wherein the cells were produced by yeast cells including the polynucleotide encoding tSPF fused to the secretion signal sequence.

**Table 1**

| Cells | 72 hours | | | | 144 hours | | | |
|---|---|---|---|---|---|---|---|---|
| | Cell growth (OD60 0) | Intracell ular (mg/L) | Intracel lular (mg/g) | Extrace llular (mg/L) | Cell growth (OD600) | Intracell ular (mg/L) | Intracell ular (mg/g) | Extracell ular (mg/L) |
| Empty vector | 20.23 | 574.98 | 69.32 | 3.99 | 23.92 | 648.03 | 66.07 | 8.73 |
| tSPF | 17.79 | 401.51 | 55.05 | 3.67 | 19.91 | 485.04 | 59.43 | 13.82 |
| Suc2-tSPF | 12.98 | 476.9 | 89.61 | 166.62 | 13.87 | 557.03 | 97.93 | 226.51 |
| Pho5-tSPF | 11.9 | 252.17 | 51.68 | 64.55 | 14.38 | 426.85 | 72.39 | 190.09 |
| MFα-tSPF | 11.44 | 210.52 | 44.88 | 4.21 | 17.16 | 551.64 | 78.42 | 43.25 |

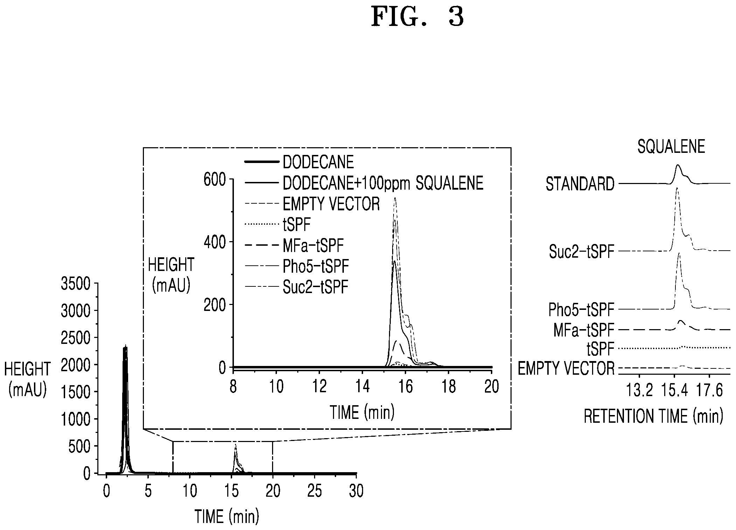

As shown in Table 1 and FIGS. 2A and 2B, the SQ strains transformed with the Suc2-tSPF, Pho5-tSPF, and MFα-tSPF fusion protein genes, respectively, showed a significant increase in the amount of extracellular squalene or in the extracellular squalene secretion ratio, compared to the control cells. FIG. 3 is a diagram showing the results of HPLC analysis of squalene produced by the SQ strains and then extracellularly secreted, wherein the SQ strains were each transformed with the Suc2-tSPF, Pho5-tSPF and MFα-tSPF fusion protein genes. The results of FIG. 3 correspond to the data of the extracellularly secreted squalene in Table 1. As shown in FIG. 3, distinct peaks of only the squalene secreted extracellularly by the Suc2-tSPF, Pho5-tSPF and MFα-tSPF fusion proteins were observed at the same retention time as the squalene standard peaks. These peaks indicate that squalene was specifically secreted extracellularly in the cells. In FIG. 3, "dodecane" and "dodecane+100 ppm squalene" are control groups, and samples of dodecane only or samples obtained by mixing dodecane and 100 ppm squalene standard were subjected to the HPLC analysis.

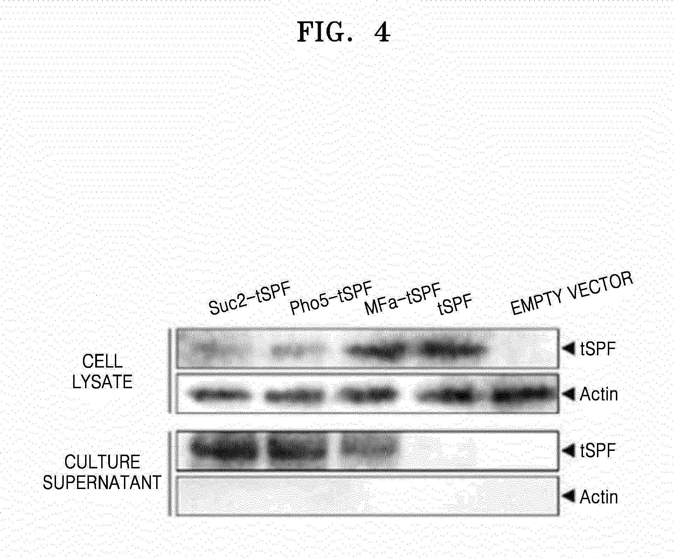

FIG. 4 is a diagram showing the results of western blot analysis of the cell lysates and the culture supernatant obtained after culturing the SQ strains that were each transformed with the Suc2-tSPF, Pho5-tSPF and MFα-tSPF fusion protein genes. To confirm the extracellular secretion of the tSPF fusion protein, yeast cells were cultured in a flask (250 m) containing 50 mL minimal medium (e.g., YSC medium; 0.67 % (w/v) yeast nitrogen base without amino acids (BD Difco, USA), 0.19 % (w/v) yeast synthetic drop-out medium supplements without uracil (Sigma-Aldrich, USA)) supplemented with 2 % (w/v) glucose while stirring at 30°C. Here, the minimal medium did not have a dodecane layer. To extract intracellular proteins from the cultured yeast cells in an amount corresponding to an OD600 value of 100, the cells were resuspended 1 mL of cell lysis buffer (1 mL RIPA buffer, Thermo Scientific, MA, USA) containing protease inhibitor (1x protease inhibitor cocktail, Roche Diagnostics, Mannheim, Germany). The suspension was homogenized by using a sonicator, for 6 minutes at 20 % amplitude and 3 second intervals. The extracellular proteins in the supernatant of the culture medium were added to a solution in which trichloroacetic acid solution was added to 50 mL of the supernatant to account for 25 %, and incubated for 30 minutes to precipitate proteins, followed by centrifugation at 4 °C for 40 minutes. The precipitated proteins were washed three times with 1 ml of cold acetone, and then resuspended in 50 µl of sterile water. The resuspended proteins were further concentrated by using 30 kDa amicon ultra centrifugal filters.

The protein samples thus prepared, i.e. the cell lysates, were mixed with 5x SDS sample buffer and separated by 10 % SDS-PAGE. The proteins separated on the SDS-PAGE gel were transferred to a PVDF membrane, and blocked by incubation for one day in TBST containing 4 % skim milk. The PVDF membrane was reacted with a solution containing primary antibodies at room temperature for 1 hour, and then washed with a TBST solution three times. As the primary antibody, an anti-actin antibody or an anti-His tag antibody was used. The fusion protein included a 6xHis tag sequence at the C- terminus. The washed PVDF membrane was reacted again in a solution containing peroxidase-conjugated anti-IgG antibodies at room temperature for 1 hour. The PVDF membrane in which the antibody-conjugated proteins were present was visualized by using the ChemiDoc imaging system.

The western blot results are shown in FIG. 4. As shown in FIG. 4, in the case of the cell lysate, bands of the Suc2-tSPF and the Pho5-tSPF hardly appeared, referring that the tSPF fused to the secretion signal sequence did not stay inside the cell, but migrated to the outside the cell. In addition, based on that an apparent band of the tSPF not fused to the secretion signal sequence appeared, it is referred that the tSPF did not migrate to the outside the cell when not fused to the secretion signal sequence. In the case of the culture supernatant, a band of the tSPF not fused to the secretion signal sequence did not appear. It refers that that the tSPF not fused to the secretion signal sequence did not migrate to the outside the cell.

### Example 2. Yeast cells with increased beta-carotene secretion ability and beta-carotene production by using the same

### (2.1) Preparation of yeast cells with enhanced biosynthetic pathway of beta-carotene

A vector containing a beta-carotene biosynthesis gene was introduced into CEN.PK2-1D yeast strain described in Section 1.1 of Example 1 to prepare yeast cells with increased beta-carotene production ability.

In detail, the crtE, crtYB, crtl, and tHMG1 genes were amplified from the pMM494 vector (Addgene, Plasmid #100539) containing expression cassettes for the beta-carotene biosynthetic genes *crtE, crtYB, crtl,* and *tHMG1,* and these genes were introduced to the sites of restriction enzymes BamHI and Sall of p415-GPD vector (SEQ ID NO: 30), to construct p415-GPD vetor including the expression cassettes for *crtE, crtYB, crtl,* and *tHMG1.* The p415-GPD vector (ATCC 87358) is a low copy number expression vector including a GPD1 promoter. CrtE, crtYB, and crt proteins have the amino acid sequences of SEQ ID NOs: 31, 32, and 33, respectively. Polynucleotides encoding *crtE, crtYB,* and *crt* have nucleotide sequences of SEQ ID NOs: 34, 35, and 36, respectively. The crtE, crtYB, and crtl proteins derived from *Xanthophyllomyces dendrohou,* and have the following activities. *CrtE* is a gene encoding geranylgeranyl diphosphate that catalyzes a conversion reaction from farnesyl diphosphate to geranylgeranyl diphosphate. *CrtYB* includes *crtB,* which encodes phytoene synthase that catalyzes a conversion reaction from geranylgeranyl diphosphate to phytoene, and *crtY,* which encodes lycopene beta-cyclase that catalyzes a conversion from lycopene to beta-carotene. *Crtl* is a gene encoding phytoene desaturase that converts phytoene to lycopene.

### (2.2) Preparation of yeast cells to which gene encoding squalene-binding protein fused to secretion signal sequence has been introduced

Through the same process as described in Section (1.2) of Example 1, the S. *cerevisiae* wild strain CEN.PK2-1D was co-transformed with the p415-BC vector manufactured in Section (1.2) and either pSEC-tSPF or pSEC-Suc2-tSPF, to manufacture yeast cells transformed with a vector expressing tSPF fused to a signal peptide.

### (2.3) Confirmation of beta-carotene production ability and beta-carotene secretion ability by recombinant yeast cells

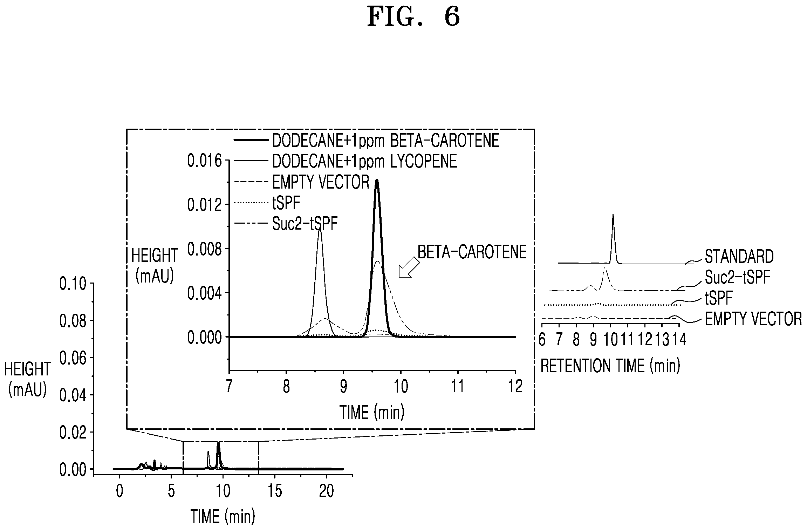

CEN.PK2-1D yeast cells, which were co-transformed with the p415-BC vector obtained in Section (2.2) and either pSEC-tSPF or pSEC-Suc2-tSPF and were able to overproduce beta-carotene, were cultured for 144 hours under the same conditions as described in Section (1.3) of Example 1. Then, the amount of beta-carotene in the cultured cells and outside the cells was measured. The amount of beta-carotene was measured in the same method as the methods for cell lysis and dodecane layer separation used for the measurement of the squalene concentration. Table 2 and FIG. 6 show the amount of beta-carotene produced by culturing the CEN.PK2-1D yeast cells co-transformed with the p415-BC vector and either pSEC-tSPF or pSEC-Suc2-tSPF.

**Table 2**

| Cells | 144 hours | | | |
|---|---|---|---|---|
| | Cell growth (OD600) | Intracellular (mg/L) | Intracellular (mg/g) | Extracellular (mg/L) |
| Empty vector | 22.58 | 9.52 | 0.84 | 0.06 |
| tSPF | 20.36 | 7.04 | 0.69 | 0.23 |
| Suc2-tSPF | 18.66 | 7.65 | 0.82 | 1.4 |

As shown in Table 2 and FIG. 6, the yeast cells transformed with the Suc2-tSPF fusion protein gene significantly increased the amount of extracellular beta-carotene or the ratio of extracellular secretion compared to control cells. In detail, the extracellular secretion of beta-carotene was increased about 23-fold compared to the control groups containing the empty vector. FIG. 6 is a diagram showing the results of HPLC analysis of beta-carotene produced by yeast cells transformed with the Suc2-tSPF fusion protein gene and then secreted extracellularly. In this regard, the results of FIG. 6 correspond to the data of the extracellularly secreted beta-carotene in Table 2, that is, the data of beta-carotene secreted in the dodecane layer. As shown in FIG. 6, peaks of beta-carotene secreted extracellularly by the Suc2-tSPF fusion protein were observed at the same retention time as the beta-carotene standard peaks. These peaks indicate that beta-carotene was specifically secreted extracellularly in the cells. In FIG. 6, "dodecane+1 ppm beta-carotene" and "dodecane+1 ppm lycopene" are control groups, and indicate a standard mixture containing 1 ppm beta-carotene in dodecane and a standard mixture containing 1 ppm lycopene in dodecane, respectively.

### Example 3. Evaluation of expansion potential to various terpenoid-based substances

To identify the ligand-binding site of tSPF, the three-dimensional crystallographic structure of tSPF (PDB ID: 4OMK) was obtained from the RCSB Protein Data Bank. For the molecular docking analysis, the A-chain of 4ONK, from which squalene and water molecules bonded in the crystal structure were removed before simulation, was used. To construct a rigid protein structure from the crystallographic structure, one possible structure was intentionally selected for each amino acid. Next, the squalene-binding active site of the A-chain was determined by using the Computer Atlas of Surface Topology of protein (CASTp). Based on the amino acid sequence of SEQ ID NO: 1, the identified residues required for the squalene binding may be L84, I103, L106, A108, L111, L112, L120, L121, K124, I151, Y153, C155, L158, H162, A167, V168, A170, Y171, F174, L175, L186, L189, F198, A201, Y202, I205, L209, T213, and I217. In this regard, the squalene-binding site was regarded to include these residues.

For the ligand selection, the following 43 terpenoids were selected from the PubChem Compound database in the sdf format: 2,3-oxidosqualene, myrcene, (z)-ocimene, linalool, geraniol, nerol, limonene, menthol, α-pinene, camphor, carvacrol, carvone, β-farnesene, nerolidol, β-bisabolene, valencene, amorphadiene, capsidiol, α-cedrane, artemisinin, phytane, phytol, camphorene, retinol, labdane, clerodane, pimarane, abietane, tigliane, kaurane, taxane, squalene, lanosterol, α-amyrin, β-amyrin, hopane, oleanolic acid, lycopene, rubixanthin, β-carotene, lutein, zeaxanthin, and astaxanthin.

In addition, glutamate and pyruvate were selected as control molecules. The downloaded sdf file was converted to a pdbqt format by Open Babel toolbox of the PyRx virtual tool. By using the information above, the molecular docking was performed with the AutoDock Vina to analyze the binding energy between tSPF and various terpenoid-based substances. The results are shown in Tables 3, 4, 5, and 6.

**Table 3**

| Name | Molecular formula | Structure | Binding energy |
|---|---|---|---|
| Pyruvate | C₃H₃O₃ | 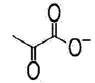 | -3.6 |
| Glutamate | C₅H₉NO₄ | 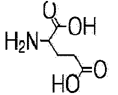 | -4.4 |
| Geraniol | C₁₀H₁₈O | 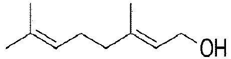 | -6.3 |
| Linalool | C₁₀H₁₈O | 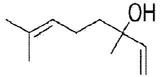 | -6.1 |
| Myrcene | C₁₀H₁₆ | 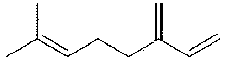 | -6.3 |
| Nerol | C₁₀H₁₈O | 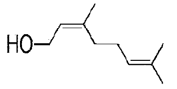 | -6.3 |
| β-Ocimene | C₁₀H₁₆ | 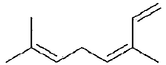 | -6.5 |
| Cam phor | C₁₀H₁₆O | 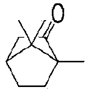 | -7.1 |
| Carvacrol | C₁₀H₁₄₀ | 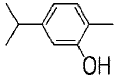 | -7.4 |
| Carvone | C₁₀H₁₄O | 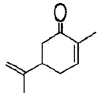 | -6.9 |
| Limonene | C₁₀H₁₆ | 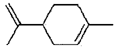 | -6.5 |

**Table 4**

| | | | |
|---|---|---|---|
| Menthol | C₁₀H₂₀O | 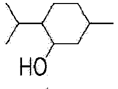 | -6.5 |
| α-Pinene | C₁₀H₁₆ | 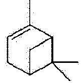 | -6.6 |
| β-Farnesene | C₁₅H₂₄ | 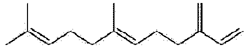 | -7.6 |
| Nerolidol | C₁₅H₂₆O | 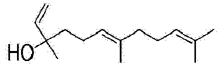 | -7.7 |
| Amorphadiene | C₁₅H₂₄ | 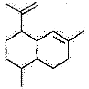 | -8.1 |
| Artemisinin | C₁₅H₂₂O₅ | 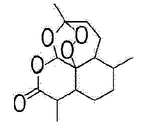 | -9.4 |
| β-Bisabolene | C₁₅H₂₄ | 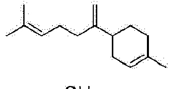 | -8.7 |
| Capsidiol | C₁₅H₂₄O₂ | 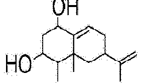 | -7.8 |
| α-Cedrane | C₁₅H₂₆ | 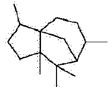 | -8.9 |
| Valencene | C₁₅H₂₄ | 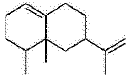 | -8.7 |
| Phytane | C₂₀H₄₂ | 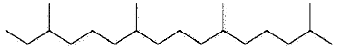 | -7.4 |
| Phytol | C₂₀H₄₀O | 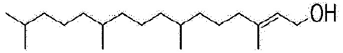 | -7.4 |

**Table 5**

| | | | |
|---|---|---|---|
| Abietane | C₂₀H₃₆ | 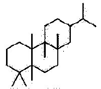 | -10.7 |
| Cam phorene | C₂₀H₃₂ | 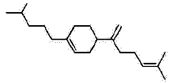 | -9.5 |
| Clerodane | C₂₀H₃₈ | 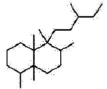 | -9.4 |
| Kaurane | C₂₀H₃₄ | 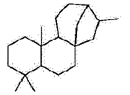 | -10.5 |
| Labdane | C₂₀H₃₈ | 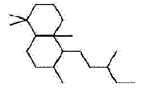 | -9.7 |
| Pimarane | C₂₀H₃₆ | 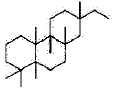 | -10.3 |
| Retinol | C₂₀H₃₀O | 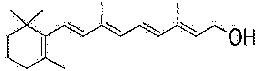 | -9.4 |
| Taxane | C₂₀H₃₆ | 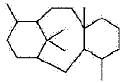 | -7.7 |
| Tigliane | C₂₀H₃₄ | 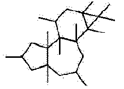 | -9.4 |
| Oxidosqualene | C₃₀H₄₉O | 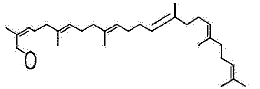 | -10.6 |
| Squalene | C₃₀H₅₀ | 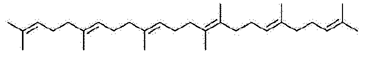 | -10.5 |
| α-Amyrin | C₃₀H₅₀O | 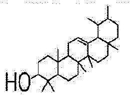 | -6.8 |

**Table 6**

| | | | |
|---|---|---|---|
| β-Amyrin | C₃₀H₅₀O | 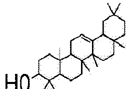 | -6.6 |
| Hopane | C₃₀H₅₂ | 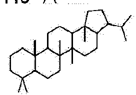 | -6.6 |
| Lanosterol | C₃₀H₅₀O | 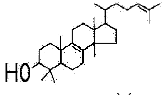 | -10.7 |
| Oleanolic acid | C₃₀H₄₈O₃ | 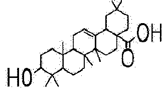 | -6.3 |
| Lycopene | C₄₀H₅₆ | 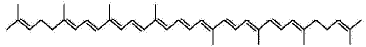 | -9.8 |
| Astaxanthin | C₄₀H₅₂O₄ | 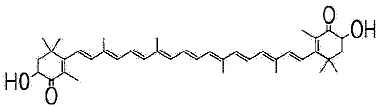 | -6.2 |
| β-Carotene | C₄₀H₅₆ | 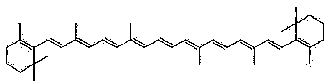 | -7.7 |
| Lutein | C₄₀H₅₆O₂ | 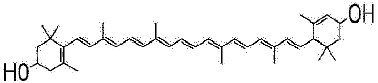 | -7.5 |
| Rubixanthin | C₄₀H₅₆0 | 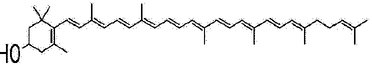 | -7.4 |
| Zeaxanthin | C₄₀H₅₆O₂ | 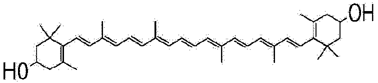 | -6.3 |

As shown in Tables 3, 4, 5, and 6, the difference in the binding energy between tSPF and each of the 43 terpenoid-based compounds was in a range of about -10.7 kcal/mol to about -6.1 kcal/mol, showing no significant difference even when compared with each of the binding energy of squalene of -10.5 kcal/mol and the binding energy of beta-carotene of -7.7 kcal/mol. These results indicate that the recombinant microorganism including the polynucleotide encoding tSPF fused to the above secretion signal sequence can be extensively applied to various terpenoid-based compounds.

## Claims

1. A recombinant microorganism comprising a polynucleotide encoding a target product-binding protein fused to a secretion signal sequence.

2. The recombinant microorganism of claim 1, wherein the recombinant microorganism has an ability to secrete the target product-binding protein fused to the secretion signal sequence.

3. The recombinant microorganism of claim 1, wherein the target product-binding protein is a lipid-binding protein.

4. The recombinant microorganism of claim 3, wherein the lipid-binding protein is selected from the group consisting of SPF, Sec14, TTP, an alpha-tocopherol transfer protein, a cellular retinal binding protein, a squid retinal binding protein, and fragments thereof having target product-binding ability.

5. The recombinant microorganism of claim 1, wherein the recombinant microorganism has an increased ability to produce the target product.

6. The recombinant microorganism of claim 1, wherein the target product is a metabolite.

7. The recombinant microorganism of claim 6, wherein the lipid is a terpenoid compound.

8. The recombinant microorganism of claim 1, wherein the target product is a compound selected from the group consisting of 2,3-oxidosqualene, myrcene, (z)-ocimene, linalool, geraniol, nerol, limonene, menthol, α-pinene, camphor, carvacrol, carvone, β-farnesene, nerolidol, β-bisabolene, valencene, amorphadiene, capsidiol, α-cedrane, artemisinin, phytane, phytol, camphorene, retinol, labdane, clerodane, pimarane, abietane, tigliane, kaurane, taxane, squalene, lanosterol, α-amyrin, β-amyrin, hopane, oleanolic acid, lycopene, rubixanthin, β-carotene, lutein, zeaxanthin, and astaxanthin.

9. The recombinant microorganism of claim 1, wherein the secretion signal sequence is one or more selected from the group consisting of Suc2, Pho5, and MFα.

10. The recombinant microorganism of claim 1, comprising a genetic modification that increases expression of a polynucleotide encoding a target product or a genetic modification that enhances a metabolic pathway for production of a target product.

11. The recombinant microorganism of claim 1, wherein the recombinant microorganism is a yeast cell.

12. The recombinant microorganism of claim 12, wherein the recombinant microorganism belongs to the genus Saccharomyces.

13. The recombinant microorganism of claim 1, wherein the target product is a compound selected from the group consisting of 2,3-oxidosqualene, myrcene, (z)-ocimene, linalool, geraniol, nerol, limonene, menthol, α-pinene, camphor, carvacrol, carvone, β-farnesene, nerolidol, β-bisabolene, valencene, amorphadiene, capsidiol, α-cedrane, artemisinin, phytane, phytol, camphorene, retinol, labdane, clerodane, pimarane, abietane, tigliane, kaurane, taxane, squalene, lanosterol, α-amyrin, β-amyrin, hopane, oleanolic acid, lycopene, rubixanthin, β-carotene, lutein, zeaxanthin, and astaxanthin,
the target product-binding protein is selected from the group consisting of SPF, Sec14, TTP, an alpha-tocopherol transfer protein, a cellular retinal binding protein, a squid retinal binding protein, and fragments thereof having target product-binding ability, and
the recombinant microorganism is a yeast cell.

14. A composition for use in producing a target product, the composition comprising a recombinant microorganism and a carrier, wherein the recombinant microorganism comprises a polynucleotide encoding a target product-binding protein fused to a secretion signal sequence.

15. A method of producing a target product, the method comprising culturing a recombinant microorganism in a medium, wherein the recombinant microorganism comprises a polynucleotide encoding a target product-binding protein fused to a secretion signal sequence.

16. The method of claim 15, wherein the culturing is performed in a medium containing a compound having high solubility of a target product as compared to solubility of the target product in the medium or water.

17. The method of claim 15, further comprising isolating the target product, wherein the isolating of the target product comprises separating a supernatant from a culture.

18. The method of claim 17, further comprising separating the target product from the supernatant.

19. The method of claim 16, comprising separating a target product from a compound layer having high solubility of the target product as compared to solubility of the target product in the medium or water.

20. The method of claim 19, wherein the target product is a compound selected from the group consisting of 2,3-oxidosqualene, myrcene, (z)-ocimene, linalool, geraniol, nerol, limonene, menthol, α-pinene, camphor, carvacrol, carvone, β-farnesene, nerolidol, β-bisabolene, valencene, amorphadiene, capsidiol, α-cedrane, artemisinin, phytane, phytol, camphorene, retinol, labdane, clerodane, pimarane, abietane, tigliane, kaurane, taxane, squalene, lanosterol, α-amyrin, β-amyrin, hopane, oleanolic acid, lycopene, rubixanthin, β-carotene, lutein, zeaxanthin, and astaxanthin,
the target product-binding protein is selected from the group consisting of SPF, Sec14, TTP, an alpha-tocopherol transfer protein, a cellular retinal binding protein, a squid retinal binding protein, and fragments thereof having target product-binding ability, and
the recombinant microorganism is a yeast cell.
